# EUROPEAN PATENT SPECIFICATION

(11) **EP 3 364 938 B1**
(45) Date of publication and mention of the grant of the patent: **01.07.2020**
(21) Application number: 15817781.6
(22) Date of filing: 14.12.2015
(51) Int. Cl.: A61K 8/34, A61K 8/49, A61Q 5/00

(54) **MIXTURES COMPRISING CLIMBAZOLE**
MISCHUNGEN MIT CLIMBAZOL
MÉLANGES COMPRENANT DU CLIMBAZOLE

(30) Priority: 23.10.2015 US 201562245583 P
(43) Date of publication of application: 29.08.2018
(73) Proprietor: Symrise AG, 37603 Holzminden (DE)
(72) Inventor: ROMEU, Xavier, 08032 Barcelona (ES); GENRICH, Florian, 37586 Dassel (DE); PILLAI, Ravikumar, Mahwah, NJ 07430 (US); LÓPEZ, Mirela, 08032 Barcelona (ES)
(74) Representative: Fabry, Bernd
(86) International application number: PCT/EP2015/079537
(87) International publication number: WO 2017/067620

(56) References cited:
- US-A1- 2010 215 775
- US-A1- 2013 136 709

## Description

### FIELD OF INVENTION

The present invention belongs to the area of cosmetics, in particular hair cosmetics and refers to a mixture comprising (*RS*)-1-(4-Chlorophenoxy)-1-imidazol-1-yl-3,3-dimethylbutan-2-one (climbazole), a mixture of two 1,2-alkane diols selected from the following combinations (a) to (d): (a) 1,2-pentanediol and 1,2- octanediol, (b) 1,2-pentanediol and 1,2- decanediol, (c) 1,2-hexanediol and 1,2- octanediol, (d) 1,2-hexanediol and 1,2- decanediol, and 2-phenoxyethanol.

### STATE OF THE ART

Dandruff is one of the most frequent skin problems, affecting more than 50% of men and women worldwide. The formation of dandruff can be triggered inter alia by the following factors:
- by functional disturbances of the sebaceous glands caused by hereditary factors,
- by diseases of internal organs or a disturbance in their functioning,
- as a side-effect of medicaments,
- by stress, strain on the nerves and psychological problems,
- by drying out of the scalp by unsuitable care products, bacteria and fungi, and
- by increased sebum production by the scalp.

Dandruff in most cases forms as a result of overproduction of horny cells. This overproduction is triggered by tiny centers of inflammation of the scalp, which are visible only under a microscope. Because of the inflammation, the horny cells of the scalp do not mature fully, so that they are shed prematurely in large cell structures - as dandruff.

A frequent cause of dandruff formation is increased colonisation with bacteria or fungi. *Malassezia* species, such as, for example, *Malassezia furfur* or *Malassezia globosa,* are of particular relevance here.

When treating increased dandruff formation, attempts are made, for example, to remove the dandruff, to reduce the overproduction of horny cells and/or to inhibit the growth of fungi. There are used, inter alia, salicylic-acid-containing therapeutic agents, antimycotics having good activity against *Malassezia* species, tar-containing externals, sulfur and/or selenium disulfide.

Inhibiting the growth of fungi of the genus *Malassezia* is one of the main methods of controlling dandruff formation. An important group of active ingredients that are cu rrently being used in the treatment of dandruff are antimycotics for topical and systemic administration from the group of the azoles, preferred anti-dandruff agents are e.g. climbazole as well as further azoles.

The document US 2010/0215775 A1 relates to the use of one, two, three or more C10-C14-alkane-1,2-diols, in particular 1,2-decanediol, 1,2-dodecanediol and/or 1,2-tetradecanediol, in the preparation of a composition for the prophylaxis and/or treatment of *Malassezia*-induced dandruff formation, as well as to cosmetic and/or dermatological preparations comprising one, two, three or more C10-C14-alkane-1,2-diols.

The document US 2013/0136709 A1 relates to certain mixtures, preparations and foodstuffs comprising 2-phenoxyethanol, and at least two different alkanediols, selected from the group consisting of 1,2-decanediol, 1,2-octanediol, 1,2-hexanediol and 1,2-pentanediol. The document further relates to the use of 2-phenoxyethanol for synergistic intensification of the antimicrobial efficacy of mixtures of at least two of the aforementioned alkanediols, a method of preservation or antimicrobial treatment of a perishable product, a method of cosmetic treatment of certain microorganisms and the use of a mixture according to the invention for the therapeutic treatment of certain microorganisms.

Climbazole (*RS*)-1-(4-chlorophenoxy)-1-imidazol-1-yl-3,3-dimethylbutan-2-one is usually used in anti-dandruff and anti-fungal products, including shampoos, lotions and conditioners. Its chemical structure and properties are similar to other fungicides such as ketoconazole and miconazole. At ambient temperature climbazole is a crystalline solid with low solubility in water, but higher solubility in alcohol, glycols, surfactants and certain perfume oils.

When using climbazole in the hair care area, which usually comprises mostly water as the main component, it is difficult to bring climbazole into solution, because often heating processes are not available technically. Thus, it was an object of the present invention to simplify the manufacture of hair care products by cold process involving climbazole. Therefore, an object of the present invention was to develop a liquid blend comprising climbazole for the manufacture of hair care products, wherein the dissolving step is eliminated from the manufacture process.

It was a further object of the present invention to provide a mixture comprising climbazole and the said component (single substance or mixture of substances) that provides improved solubility of climbazole in water, as a semi-finished product which is preferably clear, colourless or transparent and does not impact the end product's viscosity and foamability in a negative way, thus the mixture is suitable for the production of the end product, which is preferably a hair care product. Accordingly, the said mixture (or semi-finished product) should be universally suitable especially for the cold production of hair care products, where heating is not possible.

One of the main objects of the present invention relates to the stability of the said mixture (semi-finished product), which should show good storage stability until the mixture is used for the production of the end product, especially without a recrystallization of climbazole during a long-term storage time.

In addition the said mixture (semi-finished product) should if at all possible not have an unpleasant odour or should not impart an unpleasant odour to the cosmetic and product.

### DESCRIPTION OF THE INVENTION

A first object of the present invention is a mixture comprising:
(i) (*RS*)-1-(4-Chlorophenoxy)-1-imidazol-1-yl-3,3-dimethylbutan-2-one (climbazole),
(ii) a mixture of two 1,2-alkane diols selected from the following combinations (a) to (d):
   (a) 1,2-pentanediol and 1,2- octanediol,
   (b) 1,2-pentanediol and 1,2- decanediol,
   (c) 1,2-hexanediol and 1,2- octanediol,
   (d) 1,2-hexanediol and 1,2- decanediol, and
(iii) 2-phenoxyethanol.

Surprisingly, it has been observed that such a described mixture, comprising compounds (i), (ii) and (iii) achieved the above mentioned object of the present invention. In particular, it has been surprisingly observed, that although 2-phenoxyethanol which is known to be a preservative only shows moderate action activity against some micro-organisms, such as *Aspergillus brasiliensis* (ATCC 16404), but in combination with said compounds (i) and (ii) there is a high efficacy against a series of micro-organisms, including *Aspergillus brasiliensis* (ATCC 16404).

The specific combinations of 1,2-alkane diols of the invention in combination with 2-phenoxyethanol have been observed to be especially effective to keep climbazole soluble, respectively improve the solubility of climbazole for cosmetic products formulation. In particular the combination of a long-chain 1,2-alkane diol with a short-chain 1,2-alkane diol is advantageously for the improvement of the phase relationship, especially in case of an oil-in-water or water-in-oil emulsion. Thus, the solubility of climbazole can be increased through the improved phase relationship.

A further embodiment of the present invention refers to the said mixture, wherein
a) the ratio of 1,2-pentanediol and 1,2- octanediol is from 1:5 to 5:1, preferably 1:1 to 1:5 or 1:1 to 5:1;
b) the ratio of 1,2-pentanediol and 1,2- decanediol is from 1:5 to 5:1, preferably 1:1 to 1:5 or 1:1 to 5:1;
c) the ratio of 1,2-hexanediol and 1,2- octanediol is from 1:5 to 5:1, preferably 1:1 to 1:5 or 1:1 to 5:1;
d) the ratio of 1,2-hexanediol and 1,2- decanediol is from 1:5 to 5:1, preferably 1:1 to 1:5 or 1:1 to 5:1.

Within the mentioned ratios, the combinations of 1,2-alkane diols a) to d) of the invention are advantageously used to achieve (besides the ability to dissolve climbazole), to not have negative impact on viscosity and foam ability of the final cosmetic product.

In a further embodiment of the present invention the said mixture comprises:
(i) 25 w.t.% to 40 w.t.% (RS)-1-(4-Chlorophenoxy)-1-imidazol-1-yl-3,3-dimethylbutan-2-one (climbazole),
(ii) 2 w.t.% to 35 w.t.% of the total weight of the two 1,2-alkane diols,
(iii) 35 w.t.% to 55 w.t.% 2-phenoxyethanol,
wherein the percentages by weight refer to the total weight of the mixture.

Preferably the total amounts of the 1,2-alkane diols in the mixture is from 2% to 35 % by weight, preferably 3% to 30% by weight, more preferably 5% to 20.5% by weight, each based on the total weight of the total composition.

Preferably the two 1,2-alkane diols are a combination of 1,2-hexanediol and 1,2-decanediol or 1,2-pentanediol and 1,2- decanediol . This combination of 1,2-alkane diols are particularly preferred, because they are most advantageous to achieve the above mentioned object, in particular in aspects of the antimicrobial efficacy but also storage stability.

1,2- Decanediol is waxy at room temperature. A mixture of 1,2-pentanediol or 1,2-hexanediol in combination with 1,2- decanediol and 2-phenoxyethanol, which function as solubilizer as well as possess antimicrobial character, can be used with (RS)-1-(4-Chlorophenoxy)-1-imidazol-1-yl-3,3-dimethylbutan-2-one (climbazole) to prepare hair care products by a cold process.

Basically, (RS)-1-(4-Chlorophenoxy)-1-imidazol-1-yl-3,3-dimethylbutan-2-one (climbazole) is known to be antimycotic, but within the mixture of the present invention there is a synergistic effect against micro-organism, that allow an improved preservation.

Thus, an embodiment of the present invention is the used of the mixture comprising as a preservative preparation for cosmetic products.

According to the present invention the preferred alternatives of the two 1,2-alkane diols are to be applied to all subject-matters of the present invention.

A further object of the present invention is a semi-finished product for hair care products, comprising the above mentioned mixture according to the invention.

The semi-finished product in this context refers to a composition (mixture) containing the certain described ingredients (i), (ii) and (iii) from which the end-product composition (is obtained) by adding one or more components thereto, preferably cosmetically acceptable ingredients and additives. The semi-finished product is preferably a concentrate, which is typically used within the end-product in a range from 0.5 % to 5 % by weight, preferably from 0.8% by weight to 3% by weight, more preferably from 1.0% to 2% usually in combination with further ingredients and additives.

Another object of the present invention is therefore a hair care product comprising the above mentioned mixture or the semi-finished product according to the invention.

The hair care product is preferably a hair care product selected from both leave-on and wash-off category selected from: shampoo (e.g. 2-in 1 shampoo, anti-dandruff shampoo, shampoo for dry scalp, shampoo concentrate), hair conditioners, hair cure, hair coloring, hair rinse, hair styling products (gel, mousse, foam, wax, spray), relaxer, straightener, hair oil, scalp cream. Hair mask, hair tonic, serum.

Surprisingly, it has been observed that the end products, here hair care products can be manufactured with the mixture and semi-finished product of the present invention without heating and climbazole is still soluble. Further, there is no climbazole precipitate after a long-term storage time at ambient temperature.

The mixtures and semi-finished products according to the invention permit the production of hair care products, especially shampoos with good storage stability, preferably over a period of 3 months, in particular over a period of 9 months.

The storage stability of the mixtures and compositions according to the invention was characterized in that no crystallization of climbazole, demixing or phase separation was observed during the storage time of 3 or preferably 6 months in daylight and at constant storage temperature, preferably at a constant storage temperature of room temperature, preferably at a constant storage temperature of 40°C and/or 50°C, in particular preferably at a constant storage temperature of 4°C.

In addition, mixtures and compositions according to the invention have good stability, i.e. no, no substantial or, only in exceptional cases, slight, insignificant changes were observed in the aforementioned storage conditions and times with respect to viscosity, pH value, color and odor.

A further object of the present invention is a method of
(i) increasing the solubility of (*RS*)-1-(4-Chlorophenoxy)-1-imidazol-1-yl-3,3-dimethyl-butan-2-one (climbazole) and/or
(ii) decreasing the turbidity of a mixture comprising (*RS*)-1-(4-Chlorophenoxy)-1-imidazol-1-yl-3,3-dimethylbutan-2-one (climbazole) and/or
(iii) increasing transparency of a mixture comprising (*RS*)-1-(4-Chlorophenoxy)-1-imidazol-1-yl-3,3-dimethylbutan-2-one (climbazole),
   comprising the step of: mixing (*RS*)-1-(4-Chlorophenoxy)-1-imidazol-1-yl-3,3-dimethylbutan-2-one (climbazole) with
   - a mixture of two 1,2-alkane diols selected from the following combinations (a) to (d):
      (a) 1,2-pentanediol and 1,2- octanediol,
      (b) 1,2-pentanediol and 1,2- decanediol,
      (c) 1,2-hexanediol and 1,2- octanediol,
      (d) 1,2-hexanediol and 1,2- decanediol, and
   - 2-phenoxyethanol,
      to obtain a mixture according to the present invention as described above.

Another object of the present invention is the use of a combination of a mixture of two 1,2-alkane diols selected from the following combinations (a) to (d): (a) 1,2-pentanediol and 1,2- octanediol, (b) 1,2-pentanediol and 1,2- decanediol, (c) 1,2-hexanediol and 1,2-octanediol, (d) 1,2-hexanediol and 1,2- decanediol and 2-phenoxyethanol to increase the solubility of climbazole, especially in water-based or containing systems.

Preference is given to the use according to the invention, wherein the two 1,2-alkane diols are used
a) the ratio of 1,2-pentanediol and 1,2- octanediol is from 1:5 to 5:1, preferably 1:1 to 1:5 or 1:1 to 5:1;
b) the ratio of 1,2-pentanediol and 1,2- decanediol is from a) 1:5 to 5:1, preferably 1:1 to 1:5 or 1:1 to 5:1;
c) the ratio of 1,2-hexanediol and 1,2- octanediol is from a) 1:5 to 5:1, preferably 1:1 to 1:5 or 1:1 to 5:1;
d) the ratio of 1,2-hexanediol and 1,2- decanediol is from a) 1:5 to 5:1, preferably 1:1 to 1:5 or 1:1 to 5:1.

A mixture or semi-finished product according to the invention is characterized by, that climbazole is soluble and among other things:
- does not significantly impact end product's viscosity,
- does not significantly impact end product's pH value,
- shows good temperature stability,
- does not significantly impact end product's foamability,
- good color stability.

The mixture and semi-finished product according to the invention are used particularly in cosmetic preparations, especially in hair care products. Such a preparation can be provided in form of an emulsion, solution, lotion, fluid, cream, micro-emulsion, gel (e.g. hydrogel or hydrodispersion gel).

The mixture and semi-finished product according to the invention are usually used in cosmetic preparations, especially in hair care products in such a way that (RS)-1-(4-Chlorophenoxy)-1-imidazol-1-yl-3,3-dimethylbutan-2-one (climbazole) is not more than 0,5 w.t.% in the cosmetic preparation, based on the total amount of the preparation.

A hair care product preferred according to the invention is characterized in that the hair care preparation comprises:
a) about 0,5% by weight to about 1,5 % by weight of the inventive mixture, which preferably comprises 2-phenoxyethanol, climbazole, 1,2-hexanediol, 1,2-decanediol;
b) about 20 % by weight to about 30 % by weight anionic surfactant, preferably sodium laurethsulfate;
c) about 8 % by weight to about 20 % by weight amphoteric surfactant, preferably cocamidopropyl betaine;
d) about 1 % by weight to about10 % by weight emulsifier, preferably PEG-6 Caprylic/Capric glycerides;
e) about 0,5 % by weight to about 10 % by weight non-ionic surfactant, preferably decyl glucoside;
f) about 0,03 % by weight to about 2 % by weight perfume;
g) about 0,05 % by weight to about 2 % by weight Polyquaternium-10;
h) about 0,05 % by weight to about 5 % by weight salt, preferably sodium chloride, and water, wherein all compounds a-h and water add to 100% by weight of the total composition.

The hair care preparation in which the mixture, respectively the semi-finished product is formulated usually comprises further cosmetic ingredients, auxiliaries and additives, such as antidandruff agents, anti-inflammatory agents, irritation-preventing agents, irritation-inhibiting agents, antioxidants, antiseptic agents, ant-statics, binders, buffers, carrier materials, chelating agents, cell stimulants, cleansing agents, care agents, surface-active substances, emulsifiers, enzymes, essential oils, fibres, film-forming agents, fixatives, foam-forming agents, foam stabilizers, gelling agents, gel-forming agents, hair care agents, hair-setting agents, hair-straightening agents, moisture-donating agents, moisturizing substances, moisture-retaining substances, bleaching agents, strengthening agents, opacifying agents, polish, gloss agents, polymers, powders, proteins, re-oiling agents, silicones, hair promotion agents, cooling agents, stabilizers, UV-absorbing agents, UV filters, thickeners, vitamins, oils, waxes, fats, phospholipids, saturated fatty acids, mono-or polyunsaturated fatty acids, α-hydroxy acids, polyhydroxyfatty acids, liquefiers, dyestuffs, colour-protecting agents, pigments, aromas, flavouring substances, odoriferous substances, polyols, surfactants, electrolytes, organic solvents or silicone derivatives and the like as additional auxiliaries and additives.

Preferably a shampoo preparation of the present invention comprises
(a) a phase A comprising the inventive mixture of
   (i) (*RS*)-1-(4-Chlorophenoxy)-1-imidazol-1-yl-3,3-dimethylbutan-2-one (climbazole),
   (ii) a mixture of two 1,2-alkane diols selected from the following combinations (a) to (d): (a) 1,2-pentanediol and 1,2- octanediol, (b) 1,2-pentanediol and 1,2- decanediol, (c) 1,2-hexanediol and 1,2- octanediol, (d) 1,2-hexanediol and 1,2- decanedio, and
   (iii) 2-phenoxyethanol,
(b) a surfactant phase B, comprising at least one surfactant and
(c) a water phase C.

In a preferred embodiment of the invention such a shampoo preparation may consists of the compounds listed in **Table A,** but should not be limited to.

**Table A**

| **PHASE:** | **INGREDIENT:** | **Amounts [% by weight]** |
|---|---|---|
| Phase A: | Inventive mixture | 1,50 |
| Phase B: | Sodium laurethsulfate (27% in water) | 27,00 |
| | Cocamidopropyl betaine (30% in water) | 12,00 |
| | PEG-6 Caprylic/Capric glycerides | 2,50 |
| | Decyl glucoside | 2,00 |
| | Perfume | 0,50 |
| Phase C: | Water | 53,60 |
| | Polyquaternium-10 | 0,40 |
| | Sodium chloride | 0,50 |

Another object of the present invention is a process for the manufacture of a cold process shampoo, comprising the steps of:
a) mixing all ingredients of a surfactant phase B, and
b) adding water phase C step by step under well agitation to the mixture from step a),
c) adding the inventive mixture (phase A), comprising
   (i) (*RS*)-1-(4-Chlorophenoxy)-1-imidazol-1-yl-3,3-dimethylbutan-2-one (climbazole),
   (ii) a mixture of two 1,2-alkane diols selected from the following combinations (a) to (d):
      (a) 1,2-pentanediol and 1,2- octanediol,
      (b) 1,2-pentanediol and 1,2- decanediol,
      (c) 1,2-hexanediol and 1,2- octanediol,
      (d) 1,2-hexanediol and 1,2- decanediol, and
   (iii) 2-phenoxyethanol to the blend of step b), characterized in that the temperature is not elevated intentionally by external heating during the process.

A surfactant phase B is understood to comprise at least one surfactant, preferably more than one kind of surfactants.

Preferred auxiliaries and additives are anionic and/or amphoteric or zwitterionic surfactants. Typical examples of anionic surfactants are soaps, alkyl benzenesulfonates, alkanesulfonates, olefin sulfonates, alkylether sulfonates, glycerol ether sulfonates, methyl ester sulfonates, sulfofatty acids, alkyl sulfates, fatty alcohol ether sulfates, glycerol ether sulfates, fatty acid ether sulfates, hydroxy mixed ether sulfates, monoglyceride (ether) sulfates, fatty acid amide (ether) sulfates, mono- and dialkyl sulfosuccinates, mono- and dialkyl sulfosuccinamates, sulfotriglycerides, amide soaps, ether carboxylic acids and salts thereof, fatty acid isethionates, fatty acid sarcosinates, fatty acid taurides, N-acylamino acids such as, for example, acyl lactylates, acyl tartrates, acyl glutamates and acyl aspartates, alkyl oligoglucoside sulfates, protein fatty acid condensates (particularly wheat-based vegetable products) and alkyl(ether) phosphates. If the anionic surfactants contain polyglycol ether chains, they may have a conventional homolog distribution although they preferably have a narrow-range homolog distribution. Typical examples of amphoteric or zwitterionic surfactants are alkylbetaines, alkylamidobetaines, aminopropionates, aminoglycinates, imidazolinium betaines and sulfobetaines. The surfactants mentioned are all known compounds. Information on their structure and production can be found in relevant synoptic works, cf. for example J. Falbe (ed.), "Surfactants in Consumer Products", Springer Verlag, Berlin, 1987, pages 54 to 124 or J. Falbe (ed.), "Katalysatoren, Tenside und Mineralöladditive (Catalysts, Surfactants and Mineral Oil Additives)", Thieme Verlag, Stuttgart, 1978, pages 123-217. The percentage content of surfactants in the preparations may be from 0.1 to 20% by weight and is preferably from 0.5 to 5% by weight, based on the preparation.

The surfactant phase can comprises further ingredients such as perfume and further cosmetically acceptable additives.

A water phase is understood to comprise water as the main component, but can also comprise further ingredients such as salts or further cosmetically acceptable additives.

### EXAMPLE 1

Preparations comprising various combinations of the two 1,2-alkane diols and an amount of about 33.3 w.t.% of climbazole and an amount of 46.6 w.t.% have been prepared.

**Table 1**

| Formulation A-H | | | | | | | | |
|---|---|---|---|---|---|---|---|---|
| **Mixture** | **A** | **B** | **C** | **D** | **E** | **F** | **G** | **H** |
| 1,2-pentanediol | 4.3 | 12.7 | | | 7.1 | 12.5 | | |
| 1,2-hexanediol | | | 6.7 | 12.0 | | | 4.6 | 12.6 |
| 1,2- octanediol | 15.2 | 7.2 | | | | | 17.0 | 7.8 |
| 1,2-decanediol | | | 13.4 | 8.1 | 13.0 | 7.8 | | |

### EXAMPLE 2

### Storage Test

The above mixture C has been stored at the following constant temperatures for a period of 1, 3, 6 and 9 months. The results are represented in **Table 2.**

**Table 2**

| Results of the storage test | | | | |
|---|---|---|---|---|
| **Temperature 4°C** | | | | |
| | **1** | **3** | **6** | **9** |
| | White solid phase + liquid clear phase, turns to homogeneous liquid when is kept at RT | White solid phase + liquid clear phase, turns to homogeneous liquid when is kept at RT | White solid phase + liquid clear phase, turns to homogeneous liquid when is kept at RT | White solid phase + liquid clear phase, turns to homogeneous liquid when is kept at RT |

| **Temperature RT** | | | | |
|---|---|---|---|---|
| | **1** | **3** | **6** | **9** |
| | Clear, colourless | Clear, colourless | Clear, colourless | Clear, colourless |

| **Temperature 40°C** | | | | |
|---|---|---|---|---|
| | **1** | **3** | **6** | **9** |
| C | Clear, colourless | Clear, very slightly yellowish | Clear, very slightly yellowish | Clear, very slightly yellowish |

### EXAMPLE 3

### Stability in shampoo

Mixture c from Example 1 has been formulated into commonly used shampoo (see table 6). The stability of the preparations has been observed as described in example 2.

**Table 3**

| Stability in shampoo | | | | | | | |
|---|---|---|---|---|---|---|---|
| **Storage Time** | | | | | | | |
| | **1 month** | | **2 months and a half** | | | **3 months** | |
| **Temp.** | | | | **pH** | | | **pH** |
| **4°C** | White, no change | | White, no change | 7,49 | | White, no change | 7,49 |
| **RT** | White, no change | | White, no change | 7,38 | | White, no change | 7,51 |
| **40°C** | Slightly yellowish | | Slightly less yellowish, separation of the pearlizer agent | 7,52 | | Slightly less yellowish, separation of the pearlizer agent | 7,83 |

### EXAMPLE 4

### Viscosity and foamability test

Mixture c from Example 1 was formulated in a shampoo (table 6).

The pH, viscosity and the foamability of the preparation is determined as follows.

### a) Viscosity Procedure

Turn on the Viscometer. Select the Spindle according to the expected viscosity of the formulation. Screw the spindle to the viscometer. Make sure the spindle is at center of the container and there is no foam floating on the surface. Adjust the container height to reach the spindle's groove. Press 'Set Speed' button. Press 'On' button and the viscometer will start spinning. Wait for 15 seconds until CP value stabilize and note it down on the log sheet.

### b) Measurement of pH

Turn on the pH-meter. Take the electrode out of the storage container, rinse the electrode with distilled or deionised water, and dry it out with clean, absorbent laboratory wipe. Place the pH probe into the container holding the sample so that the pH glass membrane at the end of the probe is completely submerged and wait until the pH value is constant. Press the "Read" button on the pH meter and record the pH value when the measurement process ceases. Take the electrode off the solution, wash with distilled or deionised water or ethanol, dry it out with wipe and put it back into the storage container.

### c) Foam studies

The foamability and foam stability of the solutions was conducted by means of the following hand shaking method using a closed test tube containing a ½ water solution of the surfac-tant formulation and ½ free space (air) by volume. It was studied three different shampoo blends. Each shampoo formulation was tested in three different versions (first trial with 0.5% climbazole and 1% of a mixture comprising phenoxyethanol, methylparaben, ethylparaben, polyparaben and butylparaben, - second trial with 1.5% climbazole, third trial with 1.5% of a mixture comprising phenoxyethanol, methylparaben, ethylparaben, polyparaben and butylparaben, in order to study the relationship between the different active ingredients and the foamability. To determine the concentration of the shampoo blends in water to be used in order to have an optimal reading of the foam volume, several trials were conducted firstly at 1.0%, 1.5% and 2.0%. Under these test conditions the concentration of 1.5% was chosen. The foam was generated by three vigorous shakings by up-downing by hand the closed test tube (100 mL total volume) containing 50 mL of 1.5% water solution of each shampoo blend. All foam tests, including shakings, were performed three times every two minutes until obtain the values of following parameters: total volume, liquid volume and foam volume. The foamability is characterized by the volume of foam immediately after shaking (t=0), and the volume of foam, still remaining in the test tube after a certain period of time (t>0).

The results of the pH, viscosity and foamability are represented below in **Tables 4 and 5:** There are no significant changes in comparison to the standard shampoo formulations. Thus, shampoos comprising the mixture of the present invention and shampoos without the inventive mixture show nearly the same viscosity, pH and foamability.

**Table 4**

| Viscosity and pH | | | |
|---|---|---|---|
| **Formulation** | **1** | **2** | **3** |
| pH | 6.66 | 6.63 | 5.89 |
| Viscosity / cPs | 789.3 | 741.03 | 492.47 |

**Table 5**

| Foamability of mixture c in shampoo formulations 1-3 | | | |
|---|---|---|---|
| **Time / min** | **Foam Volume / mL** | | |
| **Elapsed time after shaking** | **1% of Blend** | **1.5% of Blend** | **2% of Blend** |
| **Formulation 1** | | | |
| 2,00 | 32,0 | 45,0 | 42,0 |
| 4,00 | 42,5 | 50,0 | 50,0 |
| 6,00 | 42,5 | 55,0 | 55,0 |

| **Formulation 2** | | | |
|---|---|---|---|
| 2,00 | 25,0 | 40,5 | 35,0 |
| 4,00 | 40,0 | 40,5 | 47,0 |
| 6,00 | 51,0 | 51,5 | 60,0 |

| **Formulation 3** | | | |
|---|---|---|---|
| 2,00 | 30,0 | 30,0 | 36,0 |
| 4,00 | 40,0 | 40,0 | 50,0 |
| 6,00 | 45,0 | 52,0 | 50,0 |

**Table 6**

| Mixture C in a shampoo formulation | | | | |
|---|---|---|---|---|
| **Formulation** | | **1** | **2** | **3** |
| **PHASE:** | Ingredient (INCI) | w.t.% | | |
| **A** | (*RS*)-1-(4-Chlorophenoxy)-1-imidazol-1-yl-3,3-dimethyl butan-2-one | 0,50 | 0,00 | 0,00 |
| | Phenoxyethanol, Methylparaben, Ethylparaben, Propylparaben, Butylparaben | 1,00 | 0,00 | 1,50 |
| | Mixture C | 0,00 | 1,50 | 0,00 |
| **B** | Sodium Laurethsulfate | 27,00 | 12,00 | 12,00 |
| | Cocamidopropylbetaine | 12,00 | 3,00 | 3,00 |
| | PEG-6 Caprylic/Capric Glycerides | 2,50 | 12,00 | 12,00 |
| | Decyl Glucoside | 2,00 | 2,00 | 2,00 |
| | Perfume | 0,50 | 0,50 | 0,50 |
| **C** | Water | 53,60 | 43,60 | 43,60 |
| | Polyquarternium-10 | 0,40 | 0,40 | 0,40 |
| | Sodium chloride | 0,50 | 0,50 | 0,50 |
| | Total | 100,00 | 100,00 | 100,00 |

### EXAMPLE 5

### Thaw circle test

Mixture c has been tested in a thaw circle test to determine the temperature stability of the mixture. Mixture c was tested three times, in which it has been firstly cooled down to -20°C for 24h and then brought to room temperature, where it has been kept for 24h, afterwards warming up to 40°C and kept for 24h at this temperature and then again cooled down to room temperature. This was done three times and the samples have been visually assed. The results are presented below in **Table 7.**

**Table 7**

| Results of the thaw circle test | | | |
|---|---|---|---|
| Circle | 1 | 2 | 3 |
| Temperature | | | |
| -20°C | White solid phase, turns to homogeneous liquid when is kept at RT | White solid phase, turns to homogeneous liquid when is kept at RT | White solid phase, turns to homogeneous liquid when is kept at RT |
| RT | Clear, colorless | Clear, colorless | Clear, colorless |
| +40ºC | Clear, colorless | Clear, colorless | Clear, colorless |
| RT | Clear, colorless | Clear, colorless | Clear, colorless |

### EXAMPLE 6

### Antimicrobial preservation

Mixture c from Example 1 was formulated into a shampoo (table 9) and was tested with various micro-organisms.

20 g of sample aliquots were challenged with 0.2 ml of the preparations of inoculum of each test micro-organism. After homogenizing the challenged samples were stored at 25°C. The number of viable micro-organisms was determined at 2 days, 7 days, 14 days and 28 days after the challenge.

To determine the number of viable micro-organisms suitable dilutions of the challenged sample aliquots using NNP buffer containing inactivator No.5 prepared. These samples solutions were examined by pour plate method. For enumeration of bacteria and fungi Tryptic Soy agar (TSA) and Sabouraud agar (SAB), respectively, were used. TSA plates were incubated for 3-5 days at 30-35°C, Sabouraud agar plates were incubated at 20-25°C for 5 days.

Due to the results of the microbiological examination performed the efficacy of preservation complies with the requirements of Ph. Eur. criteria A and USP category 2 for topical preparations. The results are shown in **Table 8:**

**Table 8**

| Results of antimicrobial preservation | | | | | |
|---|---|---|---|---|---|
| **Micro-organism** | **Inoculum (calculated)** | **CFU/g** | | | |
| | | **2 days** | **7 days** | **14 days** | **28 days** |
| *Escherichia coli* ATCC 8739 | 3.4 x 10⁶ | < 10 | < 10 | < 10 | < 10 |
| *Pseudomonas aeruginosa* ATCC 9027 | 2.9 x 10⁶ | < 10 | < 10 | < 10 | < 10 |
| *Stapholococcus aureus* ATCC 6538 | 2.5 x 10⁶ | < 10 | < 10 | < 10 | < 10 |
| *Candida albicans* ATCC 10231 | 2.1 x 10⁶ | < 10 | < 10 | < 10 | < 10 |
| *Aspergillus brasiliensis* ATCC 16404 | 2.2 x 10⁶ | < 10 | < 10 | < 10 | < 10 |

It has been shown that starting from an inoculated amount of > 1.0x106 CFU/g after 2 days no micro-organisms can be detected anymore (complete inhibition). Additionally no recolonization can be observed within the following 28 days.

**Table 9**

| Shampoo formulation (in % b.w.) | | |
|---|---|---|
| **PHASE** | **INGREDIENT:** | **Amount** |
| Phase A | Inventive mixture | 1,50 |
| Phase B: | Sodium laurethsulfate | 10,00 |
| | Cocamidopropylbetaine | 2,00 |
| | Cocamidopropyl betaine, Glycol Distearate, Laureth-4 | 2,00 |
| | Cocamide DEA | 2,50 |
| | Perfume | 0,50 |
| Phase C: | Water | 80,90 |
| | Guar Hydroxypropyl Trimonium chloride | 0,50 |
| | Disodium EDTA | 0,05 |
| Phase D: | Citric acid | 0,05 |
| | Total | 100,00 |

## Claims

1. A mixture comprising:
(i) (RS)-1-(4-Chlorophenoxy)-1-imidazol-1-yl-3,3-dimethylbutan-2-one (climbazole),
(ii) a mixture of two 1,2-alkane diols selected from the following combinations (a) to (d):
(a) 1,2-pentanediol and 1,2- octanediol,
(b) 1,2-pentanediol and 1,2- decanediol,
(c) 1,2-hexanediol and 1,2- octanediol,
(d) 1,2-hexanediol and 1,2- decanediol, and
(iii) 2-phenoxyethanol.

2. The mixture of claim 1, wherein
a) the ratio of 1,2-pentanediol and 1,2- octanediol is from 1:5 to 5:1;
b) the ratio of 1,2-pentanediol and 1,2- decanediol is from 1:5 to 5:1;
c) the ratio of 1,2-hexanediol and 1,2- octanediol is from 1:5 to 1:5;
d) the ratio of 1,2-hexanediol and 1,2- decanediol is from 1:5 to 1:5.

3. The mixture of claim 1 to 2, wherein the mixture comprises:
(iv) 25 w.t.% to 40 w.t.% (RS)-1-(4-Chlorophenoxy)-1-imidazol-1-yl-3,3-dimethyl-butan-2-one (climbazole),
(v) 2 w.t.% to 35 w.t.% of the total weight of the two 1,2-alkane diols,
(vi) 35 w.t.% to 55 w.t.% 2-phenoxyethanol,
wherein the percentages by weight refer to the total weight of the mixture.

4. The mixture according to any preceding claims 1 to 3, wherein the two 1,2-alkane diols is a combination of 1,2-hexanediol and 1,2-decanediol.

5. A semi-finished product for hair care products, comprising the mixture of any proceeding claims 1 to 4.

6. A hair care product comprising a mixture according to any preceding claims 1 to 4 or the semi-finished product of claim 5.

7. The hair care product of claim 6, wherein the hair care products are selected from the group consisting of: shampoo (e.g. 2-in 1 shampoo, anti-dandruff shampoo, shampoo for dry scalp, shampoo concentrate), hair conditioners, hair cure, hair coloring, hair rinse, hair styling products (gel, mousse, foam, wax, spray), relaxer, straightener, hair oil, scalp cream, hair mask, hair tonic, serum.

8. A method of
(i) increasing the solubility of (RS)-1-(4-Chlorophenoxy)-1-imidazol-1-yl-3,3-dimethylbutan-2-one (climbazole) and/or
(ii) decreasing the turbidity of a mixture comprising (RS)-1-(4-Chlorophenoxy)-1-imidazol-1-yl-3,3-dimethylbutan-2-one (climbazole) and/or
(iii) increasing transparency of a mixture comprising (RS)-1-(4-Chlorophenoxy)-1-imidazol-1-yl-3,3-dimethylbutan-2-one (climbazole),
comprising the step of: mixing (RS)-1-(4-Chlorophenoxy)-1-imidazol-1-yl-3,3-dimethylbutan-2-one (climbazole) with
- a mixture of two 1,2-alkane diols selected from the following combinations (a) to (d):
(a) 1,2-pentanediol and 1,2- octanediol,
(b) 1,2-pentanediol and 1,2- decanediol,
(c) 1,2-hexanediol and 1,2- octanediol,
(d) 1,2-hexanediol and 1,2- decanediol, and
- 2-phenoxyethanol,
to obtain a mixture according to any claims 1 to 4 or a semi-finished product according to claim 5.

9. The use of a combination of
(i) a mixture of two 1,2-alkane diols selected from the following combinations (a) to (d):
(a) 1,2-pentanediol and 1,2- octanediol,
(b) 1,2-pentanediol and 1,2- decanediol,
(c) 1,2-hexanediol and 1,2- octanediol,
(d) 1,2-hexanediol and 1,2- decanediol, and
(ii) 2-phenoxyethanol
to increase the solubility of (RS)-1-(4-Chlorophenoxy)-1-imidazol-1-yl-3,3-dimethyl-butan-2-one (climbazole).

10. The use of claim 9, wherein
(a) the ratio of 1,2-pentanediol and 1,2- octanediol is from 1:5 to 1:5;
(b) the ratio of 1,2-pentanediol and 1,2- decanediol is from 1:5 to 1:5;
(c) the ratio of 1,2-hexanediol and 1,2- octanediol is from 1:5 to 1:5;
(d) the ratio of 1,2-hexanediol and 1,2- decanediol is from 1:5 to 1:5.

11. A process for the manufacture of a cold process shampoo, comprising the steps of:
(a) mixing all ingredients of a surfactant phase B, and
(b) adding water phase C step by step under well agitation to the mixture from step a)
(c) adding the inventive mixture (phase A), comprising
(i) (*RS*)-1-(4-Chlorophenoxy)-1-imidazol-1-yl-3,3-dimethylbutan-2-one (climbazole),
(ii) a mixture of two 1,2-alkane diols selected from the following combinations (a) to (d):
(a) 1,2-pentanediol and 1,2- octanediol,
(b) 1,2-pentanediol and 1,2- decanediol,
(c) 1,2-hexanediol and 1,2- octanediol,
(d) 1,2-hexanediol and 1,2- decanediol, and
(iii) 2-phenoxyethanol
to the blend of step b), **characterized in that** the temperature is not elevated intentionally by external heating during the process.

## Patentansprüche

1. Mischung aus:
(i) (RS)-1-(4-Chlorphenoxy)-1-imidazol-1-yl-3,3-dimethylbutan-2-on (Climbazol),
(ii) ein Gemisch aus zwei 1,2-Alkandiolen, ausgewählt aus den folgenden Kombinationen (a) bis (d):
(a) 1,2-Pentandiol und 1,2-Octandiol,
(b) 1,2-Pentandiol und 1,2-Decandiol,
(c) 1,2-Hexandiol und 1,2-Octandiol,
(d) 1,2-Hexandiol und 1,2-Decandiol und
(iii) 2-Phenoxyethanol.

2. Die Mischung nach Anspruch 1, wobei
(a) das Verhältnis von 1,2-Pentandiol und 1,2-Octandiol von 1:5 und 5:1 beträgt
(b) das Verhältnis von 1,2-Pentandiol und 1,2-Decandiol von 1:5 bis 5:1 beträgt;
(c) das Verhältnis von 1,2-Hexandiol und 1,2-Octandiol von 1:5 bis 1:5 beträgt;
(d) das Verhältnis von 1,2-Hexandiol und 1,2-Decandiol von 1:5 bis 1:5 beträgt.

3. Die Mischung nach den Ansprüchen 1 bis 2, wobei die Mischung umfasst:
(iv) 25 Gew.-T.-% bis 40 Gew.-T.-% (RS)-1-(4-Chlorphenoxy)-1-imidazol-1-yl-3,3-dimethylbutan-2-on (Climbazol),
(v) 2 Gew.-% bis 35 Gew.-% des Gesamtgewichts der beiden 1,2-Alkan-Diole,
(vi) 35 Gew.-T.-% bis 55 Gew.-%.-% 2-Phenoxyethanol,
wobei sich die Gewichtsprozente auf das Gesamtgewicht der Mischung beziehen.

4. Die Mischung nach den vorhergehenden Ansprüchen 1 bis 3, wobei die beiden 1,2-Alkandiole eine Kombination von 1,2-Hexandiol und 1,2-Decandiol darstellen.

5. Ein Halbfertigprodukt für Haarpflegeprodukte, umfassend die Mischung nach einem der Ansprüche 1 bis 4.

6. Ein Haarpflegeprodukt, das eine Mischung nach den vorstehenden Ansprüchen 1 bis 4 oder das Halbfertigprodukt nach Anspruch 5 umfasst.

7. Haarpflegeprodukt nach Anspruch 6, wobei die Haarpflegeprodukte ausgewählt sind aus der Gruppe bestehend aus: Shampoo (z.B. 2-in-1-Shampoo, Anti-Schuppen-Shampoo, Shampoo für trockene Kopfhaut, Shampoo-Konzentrat), Haarspülungen, Haarkuren, Haarfärbemittel, Haarspülungen, Haarstylingprodukten (Gel, Schaum, Schaum, Wachs, Spray), Entspannungsmitteln, Glättemitteln, Haarölen, Kopfhautcremes, Haarmasken, Haartonikum und Seren.

8. Verfahren zur
(i) Erhöhung der Löslichkeit von (RS)-1-(4-Chlorphenoxy)-1-imidazol-1-yl-3,3-dime-thyl-butan-2-on (Climbazol) und/oder
(ii) Verminderung der Trübung einer Mischung, die (RS)-1-(4-Chlorphenoxy)-1-imidazol-1-yl-3,3-dimethylbutan-2-on (Climbazol) und/oder
(iii) Erhöhung der Transparenz einer Mischung, die (RS)-1-(4-Chlorphenoxy)-1-imidazol-1-yl-3,3-dimethylbutan-2-on (Climbazol) enthält,
umfassend den Schritt: Mischen von (RS)-1-(4-Chlorphenoxy)-1-imidazol-1-yl-3,3-dimethyl-butan-2-on (Climbazol) mit
- einer Mischung aus zwei 1,2-Alkandiolen, ausgewählt aus den folgenden Kombinationen (a) bis (d):
(a) 1,2-Pentandiol und 1,2-Octandiol,
(b) 1,2-Pentandiol und 1,2-Decandiol,
(c) 1,2-Hexandiol und 1,2-Octandiol,
(d) 1,2-Hexandiol und 1,2-Decandiol und
- 2-Phenoxyethanol,
um eine Mischung nach den Ansprüchen 1 bis 5 oder ein Halbfertigprodukt nach Anspruch 6 zu erhalten.

9. Verwendung einer Kombination aus
(i) einem Gemisch aus zwei 1,2-Alkandiolen, ausgewählt aus den folgenden Kombinationen (a) bis (d):
(a) 1,2-Pentandiol und 1,2-Octandiol,
(b) 1,2-Pentandiol und 1,2-Decandiol,
(c) 1,2-Hexandiol und 1,2-Octandiol,
(d) 1,2-Hexandiol und 1,2-Decandiol und
(ii) 2-Phenoxyethanol
zur Erhöhung der Löslichkeit von (RS)-1-(4-Chlorphenoxy)-1-imidazol-1-yl-3,3-dimethylbutan-2-on (Climbazol).

10. Verwendung nach Anspruch 9, wobei
(a) das Verhältnis von 1,2-Pentandiol und 1,2-Octandiol liegt zwischen 1:5 und 1:5;
(b) das Verhältnis von 1,2-Pentandiol und 1,2-Decandiol von 1:5 bis 1:5 beträgt;
(c) das Verhältnis von 1,2-Hexandiol und 1,2-Octandiol von 1:5 bis 1:5 beträgt; (d) das Verhältnis von 1,2-Pentandiol und 1,2-Decandiol von 1:5 bis 1:5 beträgt;
(d) das Verhältnis von 1,2-Hexandiol und 1,2-Decandiol von 1:5 bis 1:5 beträgt; (e) das Verhältnis von 1,2-Hexandiol und 1,2-Octandiol von 1:5 bis 1:5 beträgt.

11. Verfahren zur Herstellung eines nach einem Kaltverfahren hergestellten Shampoos, umfassend die Schritte:
(a) Mischen aller Bestandteile eines Tensids der Phase B, und
(b) schrittweise Zugabe der Wasserphase C unter Rühren zu der Mischung aus Schritt (a)
(c) Hinzufügen der erfinderischen Mischung (Phase A), umfassend
(i) (RS)-1-(4-Chlorphenoxy)-1-imidazol-1-yl-3,3-dimethylbutan-2-on (Climbazol),
(ii) ein Gemisch aus zwei 1,2-Alkandiolen, ausgewählt aus den folgenden Kombinationen (a) bis (d):
(a) 1,2-Pentandiol und 1,2-Octandiol,
(b) 1,2-Pentandiol und 1,2-Decandiol,
(c) 1,2-Hexandiol und 1,2-Octandiol,
(d) 1,2-Hexandiol und 1,2-Decandiol und
(iii) 2-Phenoxyethanol
zu der Mischung von Schritt b), **dadurch gekennzeichnet, dass** die Temperatur während des Prozesses nicht vorsätzlich durch externe Erwärmung erhöht wird.

## Revendications

1. Un mélange comprenant :
(i) (RS)-1-(4-Chlorophénoxy)-1-imidazol-1-yl-3,3-diméthylbutan-2-one (climbazole),
(ii) un mélange de deux 1,2-alcanediols choisis parmi les combinaisons suivantes (a) à (d) :
(a) 1,2-pentanediol et 1,2- octanediol,
(b) le 1,2-pentanediol et le 1,2- décanediol,
(c) 1,2-hexanediol et 1,2- octanediol,
(d) le 1,2-hexanediol et le 1,2- décanediol, et
(iii) 2-phénoxyéthanol.

2. Le mélange de la revendication 1, dans lequel
(a) le rapport entre le 1,2-pentanediol et le 1,2- octanediol est de 1:5 à 5:1 ;
(b) le rapport entre le 1,2-pentanediol et le 1,2- décanediol est compris entre 1:5 et 5:1 ;
(c) le rapport entre le 1,2-hexanediol et le 1,2- octanediol est compris entre 1:5 et 1:5 ;
(d) le rapport entre le 1,2-hexanediol et le 1,2- décanediol est compris entre 1:5 et 1:5.

3. Mélange selon la revendication 1 à 2, **caractérisé en ce que** le mélange comprend : a) du 1,2-hexanediol et du 1,2- décanediol dans un rapport de 1:5 à 1:5 ; b) du 1,2-hexanediol et du 1,2- décanediol dans un rapport de 1:5 à 1:5 :
(i) 25 % en poids à 40 % en poids de (RS)-1-(4-Chlorophénoxy)-1-imidazol-1-yl-3,3-diméthyl¬butan-2-one (climbazole),
(ii) 2 % à 35 % en poids du poids total des deux diols 1,2-alcanes,
(iii) 35 % à 55 % en poids de 2-phénoxyéthanol,
où les pourcentages en poids se rapportent au poids total du mélange.

4. Le mélange selon les revendications 1 à 3 précédentes, dans lequel les deux 1,2-alcanediols sont une combinaison de 1,2-hexanediol et de 1,2-décanediol.

5. Produit semi-fini pour produits de soins capillaires, comprenant le mélange selon l'une quelconque des revendications précédentes 1 à 4.

6. Produit de soins capillaires comprenant un mélange selon l'une quelconque des revendications précédentes 1 à 4 ou le produit semi-fini selon la revendication 5.

7. Produit de soins capillaires selon la revendication 6, dans lequel les produits de soins capillaires sont choisis dans le groupe constitué par : un shampooing (par exemple un shampooing 2 en 1, un shampooing antipelliculaire, un shampooing pour cuir chevelu sec, un shampooing concentré), des conditionneurs de cheveux, un traitement capillaire, une coloration capillaire, un rinçage capillaire, des produits de coiffage (gel, mousse, mousse, cire, spray), un défrisant, un lisseur, une huile capillaire, une crème pour le cuir chevelu, un masque capillaire, un tonique capillaire, un sérum.

8. Une méthode de
(i) augmenter la solubilité du (RS)-1-(4-Chlorophénoxy)-1-imidazol-1-yl-3,3-dime¬thyl-butan-2-one (climbazole) et/ou
(ii) diminuer la turbidité d'un mélange comprenant du (RS)-1-(4-Chlorophénoxy)-1-imidazol-1-yl-3,3-diméthylbutan-2-one (climbazole) et/ou
(iii) l'augmentation de la transparence d'un mélange comprenant du (RS)-1-(4-Chlorophénoxy)-1-imidazol-1-yl-3,3-diméthylbutan-2-one (climbazole),
comprenant l'étape consistant à : mélanger la (RS)-1-(4-Chlorophénoxy)-1-imidazol-1-yl-3,3-diméthyl-butan-2-one (climbazole) avec
- un mélange de deux 1,2-alcanediols choisis parmi les combinaisons suivantes (a) à (d) :
(a) 1,2-pentanediol et 1,2- octanediol,
(b) le 1,2-pentanediol et le 1,2- décanediol,
(c) 1,2-hexanediol et 1,2- octanediol,
(d) le 1,2-hexanediol et le 1,2- décanediol, et
- 2-phénoxyéthanol,
pour obtenir un mélange selon les revendications 1 à 5 ou un produit semi-fini selon la revendication 6.

9. L'utilisation d'une combinaison de
(i) un mélange de deux 1,2-alcanediols choisis parmi les combinaisons suivantes (a) à (d) :
(a) 1,2-pentanediol et 1,2- octanediol,
(b) le 1,2-pentanediol et le 1,2- décanediol,
(c) 1,2-hexanediol et 1,2- octanediol,
(d) le 1,2-hexanediol et le 1,2- décanediol, et
(ii) 2-phénoxyéthanol
pour augmenter la solubilité de la (RS)-1-(4-Chlorophénoxy)-1-imidazol-1-yl-3,3-diméthylbutan-2-one (climbazole).

10. Utilisation de la revendication 9, dans laquelle
(a) le rapport entre le 1,2-pentanediol et le 1,2- octanediol est de 1:5 à 1:5 ;
(b) le rapport entre le 1,2-pentanediol et le 1,2- décanediol est compris entre 1:5 et 1:5 ;
(c) le rapport entre le 1,2-hexanediol et le 1,2- octanediol est compris entre 1:5 et 1:5 ;
(d) le rapport entre le 1,2-hexanediol et le 1,2- décanediol est compris entre 1:5 et 1:5.

11. Procédé de fabrication d'un shampooing pour procédé à froid, comprenant les étapes suivantes
(a) le mélange de tous les ingrédients d'une phase B d'agent tensioactif, et
(b) l'ajout de la phase aqueuse C, étape par étape, sous agitation, au mélange de l'étape a)
(c) l'ajout du mélange inventif (phase A), comprenant
(i) (RS)-1-(4-Chlorophénoxy)-1-imidazol-1-yl-3,3-diméthylbutan-2-one (climbazole),
(ii) un mélange de deux 1,2-alcanediols choisis parmi les combinaisons suivantes (a) à (d) :
(a) 1,2-pentanediol et 1,2- octanediol,
(b) le 1,2-pentanediol et le 1,2- décanediol,
(c) 1,2-hexanediol et 1,2- octanediol,
(d) le 1,2-hexanediol et le 1,2- décanediol, et
(iii) 2-phénoxyéthanol
au mélange de l'étape b), **caractérisé en ce que** la température n'est pas élevée intentionnellement par un chauffage externe pendant le processus.
